# EUROPÄISCHE PATENTSCHRIFT

(11) **EP 4 016 045 B1**
(45) Veröffentlichungstag und Bekanntmachung des Hinweises auf die Patenterteilung: **05.02.2025**
(21) Anmeldenummer: 20215095.9
(22) Anmeldetag: 17.12.2020
(51) Int. Cl.: G01N 1/30, G01N 33/483

(54) **VERFAHREN ZUR OPTISCHEN KLÄRUNG EINER GEWEBEPROBE MIT EINEM EINBETTUNGSMEDIUM**
METHOD FOR OPTICALLY CLARIFYING A TISSUE SAMPLE WITH AN EMBEDDING MEDIUM
PROCÉDÉ DE CLARIFICATION OPTIQUE D'UN ÉCHANTILLON TISSULAIRE COMPRENANT UN MILIEU D'ENROBAGE

(43) Veröffentlichungstag der Anmeldung: 22.06.2022
(73) Patentinhaber: MobiCron GmbH, 37083 Göttingen (DE)
(72) Erfinder: DIEKMANN, Stephan, 86911 Dießen (DE); WOUTERS-BUNT, Fred, 37085 Göttingen (DE)
(74) Vertreter: Patentanwälte Olbricht Buchhold Keulertz

(56) Entgegenhaltungen:
- EP-A2- 1 066 824
- KLAUS BECKER ET AL: "Chemical Clearing and Dehydration of GFP Expressing Mouse Brains", PLOS ONE, vol. 7, no. 3, 30 March 2012 (2012-03-30), pages e33916, XP055500434, DOI: 10.1371/journal.pone.0033916
- KATROLIA S P ET AL: "STUDIES ON ANTIFUNGAL AGENTS: AROMATIC ACID HYDRAZONES OF O. VANILLIN, O. VERATRALDEHYDE, 5-BROMO-O. VANILLIN AND BOURBONAL", HINDUSTAN ANTIBIOTICS BULLETIN, XX, XX, vol. 31, no. 3/04, 1 August 1989 (1989-08-01), pages 65 - 70, XP008017285, ISSN: 0018-1935

## Beschreibung

Die Erfindung betrifft ein Verfahren gemäß dem Oberbegriff von Anspruch 1, ein Kit zur Präparation von biologischen Gewebeproben gemäß Anspruch 10 und eine Verwendung gemäß Anspruch 12.

Transparente biologische Gewebeproben werden benötigt, um die Gewebeproben zum Beispiel mittels Lichtblattmikroskopie dreidimensional abbilden zu können. Um die Transparenz von biologischen Präparaten zu erreichen, sind insbesondere Häm-Gruppen des Blutfarbstoffs Hämoglobin und Lipide aus den biologischen Präparaten zu entfernen. Bei den "entwässernden" Verfahren wird das Gewebe mit verschiedenen Mischungen eines mit Wasser mischbaren organischen Lösungsmittels und Wasser behandelt. Die Behandlung wird mit einem aufsteigenden Anteil des organischen Lösungsmittels durchgeführt, um das Wasser vollständig aus dem Gewebe zu entfernen. Hier gibt es mehrere Möglichkeiten, wie z.B. Tetrahydrofuran, Methanol, Isopropanol, tert. Butanol und Ethanol. Für das pathologische Gewebeclearing ist Ethanol zurzeit das meist genutzte Entwässerungsmedium. Das Endresultat aller "entwässernden" Verfahren ist eine wasserfreie Probe.

Der letzte Schritt der verschiedenen Clearingverfahren ist das Angleichen des Brechungsindexes an den Brechungsindex des zu mikroskopierenden Gewebes. Der Brechungsindex von entwässertem Gewebe beträgt laut Spalteholz ("Über das Durchsichtigmachen von menschlichen und tierischen Präparaten", Verlag von S. Hirzel, 1911, Deutsches Reichs-Patent Nr. 229044) n = 1.547 für Knochen. Der Brechungsindex anderer Gewebe lässt sich anhand der von ihm empirisch ermittelten optimalen Mischungen auf ca. n = 1.551 abschätzen. Ein dermaßen hoher Brechungsindex erfordert den Einsatz aromatischer Verbindungen, die generell nicht mit Wasser mischbar sind. Spalteholz nutzte für seine Untersuchungen Mischungen aus Wintergrünöl (Methylsalicylat) und Benzylbenzoat oder Isosafrol in einem den unterschiedlichen Geweben angepassten Mischungsverhältnis.

Eine Variante des Spalteholz'sche Verfahrens ist aus Dodt, Leischner, Schierloh, Jährling, Mauch, Deininger, Deussing, Eder, Zieglgänsberger, Becker "Ultramicroscopy: three-dimensional visualization of neuronal networks in the whole mouse brain". Nat Methods. 2007;4(4):331-6.) bekannt, bei dem "Murray's clear" (eine 2:1 Mischung aus Benzylbenzoat und Benzylalkohol mit einem Brechungsindex von n = 1.559) als Einbettungsmedium eingesetzt wird. Eine weitere Variante ist aus Becker, Jährling, Saghafi, Weiler, Dodt H "Chemical Clearing and Dehydration of GFP Expressing Mouse Brains" (2012) PLoS ONE 7(3): e33916. https://doi.org/10.1371/journal.pone.0033916 bekannt. Es werden Einbettungsmedien von Spalteholz sowie einige weitere aromatische Verbindungen getestet. Dibenzylether (DBE, Brechungsindex n = 1.562) stellte sich als am besten geeignete Verbindung heraus und hat sich zum de facto Goldstandard beim Gewebeclearing etabliert. Der Vorteil des DBE beruht auch darauf, dass es in der Nutzung einfach ist, weil die Lösung nicht angemischt und dann auf den Brechungsindex überprüft werden muss; es kann direkt als Reinstoff genutzt werden.

Aus WO 2017/093323 A1 ist die Verwendung von Ethylcinnamat (ECi) und verwandter Cinnamylester als ungiftige Alternativen zu den bis dahin gängigen Einbettungsmedien bekannt. Es zeigt eine geringere akute und chronische Toxizität als die anderen zur Gewebebehandlung eingesetzten aromatischen Verbindungen, wie Dibenzylether, Benzylalkohol oder Benzylbenzoat. Da es als reine Substanz den gewünschten Brechungsindex erreicht, entfällt ein Mischschritt und damit ein Arbeitsschritt und eine mögliche Fehlerquelle.

Der Auswahl an flüssigen Reinstoffen, die sich als Einbettungsmedien für entwässerte Proben eignen, beschränkt sich somit zurzeit auf die zwei Verbindungen Dibenzylether und Ethylcinnamat. Beide Substanzen haben aber jeweils Nachteile: Dibenzylether ist giftig. Ethylcinnamat hat zwar eine geringe Toxizität, sein Schmelzpunkt liegt aber bei 6- 9°C, so dass die mit Ethylcinnamat geklärten Proben nicht im Kühlschrank aufbewahrt werden können, da das Einbettungsmedium dort auskristallisiert. Ethylcinnamat hat außerdem einen vergleichsweise hohen Dampfdruck.

Der Erfindung liegt die Aufgabe zugrunde, ein Verfahren zur Herstellung transparenter Gewebeprobe eines biologischen Gewebes zur lichtmikroskopischen Untersuchung bereitzustellen, das die vorstehenden Nachteile überwindet und insbesondere mit weniger Aufwand verbunden ist, als Mischungen als Einbettungsmedium herzustellen, Einbettungsmedien erhöhter Toxizität vermeidet und das gekühlte Lagern der entwässerten Gewebeproben erlaubt.

Die Aufgabe wird erfindungsgemäß gelöst durch ein Verfahren gemäß Patentanspruch 1. Die Aufgabe wird weiterhin gelöst durch ein Kit gemäß Patentanspruch 10 und die Verwendung gemäß Patentanspruch 12.

Weitere Ausführungsformen sind Gegenstand der Unteransprüche oder nachfolgend beschrieben.

Das erfindungsgemäße Verfahren zur Herstellung transparenter Gewebeproben eines biologischen Gewebes zur lichtmikroskopischen Untersuchung umfasst die Schritte:
a) Entwässern der Gewebeprobe mit einem entwässernden Lösungsmittel und
b) Klären der entwässerten Gewebeprobe durch Einlegen in ein flüssiges Einbettungsmedium mit einem zum Gewebe passenden Brechungsindex.

Das Einbettungsmedium enthält einen Benzaldehyd-Anisol-Ether, bevorzugt ausgewählt aus 3-Methoxybenzaldehyd, 4-Methoxybenzaldehyd, 2-Hydroxy-5-methoxybenzaldehyd oder 4-Ethoxy-benzaldehyd.

Das erfindungsgemäße Verfahren dient dazu, die optische Transparenz einer biologischen bzw. humanen Gewebeprobe für die Lichtmikroskopie zu erreichen. Das biologische Gewebe ist beispielweise humanes Gewebe oder tierisches Gewebe.

Der Klärungsschritt des Clearingverfahrens ist das Angleichen des Brechungsindexes an den Brechungsindex des zu mikroskopierenden Gewebes. Die Gewebeprobe wird hierzu in eine Brechungsindex-angleichende Lösung, das Einbettungsmedium, überführt. Das Einbettungsmedium muss mit dem Lösungsmittel des Entwässerungsschritts mischbar sein.

Das Einbettungsmedium enthält in einer Ausführungsform 10 bis 100 Vol.-% des Benzaldehyd-Ethers und 0 bis 90 Vol.-% eines optisch geeigneten, inerten organischen Lösungsmittels mit einem Brechungsindex von etwa 1,3, bevorzugt von etwa 1,5. Beispielsweise enthält das Einbettungsmedium 90 bis 100 Vol.-% des Benzaldehyd-Ethers.

Das Einbettungsmedium enthält in einer anderen Ausführungsform 10 bis 100 Vol.-% des Benzaldehyd-Ethers und 0 bis 90 Vol.-% eines optisch geeigneten, inerten organischen Lösungsmittels mit einem Brechungsindex von etwa 2,0, bevorzugt von etwa 1,65. Beispielsweise enthält das Einbettungsmedium 90 bis 100 Vol.-% des Benzaldehyd-Ethers.

Alle erfindungsgemäßen Prozentangaben erfolgen in Volumenprozent (Vol.-%).

In der bevorzugten Ausführungsform besteht das Einbettungsmedium aus einem der erfindungsgemäßen Benzaldehyd-Anisol-Ether, d.h. der Benzaldehyd-Anisol-Ether wird als Reinsubstanz eingesetzt. Als Reinsubstanz wird dabei der Benzaldehyd-Anisol-Ether in der technisch verfügbaren Reinheit verstanden.

Der Benzaldehyd-Ether ist ein Benzaldehyd-Anisol-Ether. Bevorzugt ist der Benzaldehyd-Anisol-Ether ausgewählt aus 3-Methoxybenzaldehyd (meta-Anisaldehyd; CAS Nr. 591-31-1), 4-Methoxybenzaldehyd (para-Anisaldehyd; CAS Nr. 123-11-5), 2-Hydroxy-5-methoxybenzaldehyd (6-Hydroxy-m-Anisaldehyd; CAS Nr. 672-13-9) oder 4-Ethoxy-benzaldehyd (HomoAnisaldehyd; CAS Nr. 10031-82-0). 3-Methoxybenzaldehyd weist als technisches Produkt eine Reinheit von ca. 97,0 % auf. 4-Methoxybenzaldehyd weist als technisches Produkt eine Reinheit von ca. 97,0 % auf. 2-Hydroxy-5-methoxybenzaldehyd weist als technisches Produkt eine Reinheit von ca. 98,0 % auf. 4-Ethoxy-benzaldehyd weist als technisches Produkt eine Reinheit von ca. 99 % auf.

Der Entwässerungsschritt a) dient dazu, eine wasserfreie Gewebeprobe zu erhalten. Die Gewebeprobe wird in einer Ausführungsform in mehreren Durchgängen bevorzugt mit verschiedenen Dehydratisierungs-Zusammensetzungen mit einer absteigenden Wasserreihe behandelt, d.h. Mischungen mit einem aufsteigenden Anteil eines mit Wasser mischbaren organischen Lösungsmittels, um das Wasser aus dem Gewebe zu entfernen. Als entwässerndes Medium werden Alkohole, Ketone oder Ether eingesetzt. Geeignete entwässernde Lösungsmittel sind z.B. Ethanol, Methanol, Isopropanol, Tert. Butanol (IUPAC: 2-Methylpropan-2-ol), Tetrahydrofuran oder Aceton.

Das Entwässerungsmedium hat dabei folgende Eigenschaften: 1. es mischt sich vollständig mit Wasser, damit über eine aufsteigende Konzentrationsreihe dem Gewebe schrittweise das Wasser und das Fixationsmittel entzogen werden kann und 2. es mischt sich vollständig mit dem Einbettungsmedium, welches den Brechungsindex an den des entwässerten Gewebes angleicht.

In einer alternativen Ausführungsform erfolgt der Entwässerungsschritt a) mit 2,2-Dimethoxypropan (DMP), das durch eine chemische Reaktion mit dem im Gewebe enthaltenen Wasser dieses Wasser aus dem Gewebe entfernt.

Beim erfindungsgemäßen Verfahren umfasst der Entwässerungsschritt a) in einer Ausführungsform zum Erhalt einer wasserfreien Gewebeprobe die Anwendung von Dehydratisierungs-Zusammensetzungen
- aus wässrigem Ethanol mit steigenden Konzentrationen von Ethanol, wobei die Ethanolkonzentrationen der Dehydratisierungs-Zusammensetzungen von 30% bis 100 Vol-% reichen oder
- aus wässrigem Tetrahydrofuran mit steigenden Konzentrationen von Tetrahydrofuran, wobei die Tetrahydrofurankonzentration der Dehydratisierungs-Zusammensetzungen von 30% bis 100 Vol-% reichen oder
- aus wässrigem Methanol mit steigenden Konzentrationen von Methanol, wobei die Methanolkonzentrationen der Dehydratisierungs-Zusammensetzungen von 30% bis 100 Vol-% reichen oder
- aus einer wässrigen Mischung eines anderen Alkohols, Ketons oder Ethers, wobei die Lösungsmittelkonzentration der Dehydratisierungs-Zusammensetzungen von 30% bis 100 Vol-% reichen.

In einer alternativen Ausführungsform des erfindungsgemäßen Verfahrens wird der Entwässerungsschritt a) zum Erhalt einer Gewebeprobe mit einem Restwassergehalt >2 % unter Anwendung von Dehydratisierungs-Zusammensetzungen
- aus wässrigem Ethanol mit steigenden Konzentrationen von Ethanol, wobei die Ethanolkonzentrationen der Dehydratisierungs-Zusammensetzungen von 50% bis 98 Vol-%, bevorzugt 70 % bis 98% , besonders bevorzugt 75 bis 98 Vol.% reichen oder
- aus einer wässrigen Mischung eines anderen Alkohols, Ketons oder Ethers, wobei die Lösungsmittelkonzentration der Dehydratisierungs-Zusammensetzungen von 50% bis 98 Vol-%, bevorzugt 70 % bis 98% , besonders bevorzugt 75 bis 98 Vol.% reichen.

Die eingesetzten Dehydratisierungs-Zusammensetzungen enthalten bevorzugt, auch in der letzten Stufe des Entwässerungsschritts noch 2-30 Vol.-% Wasser. In dieser Ausführungsform kann beispielsweise auch vergällter Ethanol mit einem Restwassergehalt von z.B. 4-6 Vol.-% eingesetzt werden.

In einer weiteren alternativen Ausführungsform des erfindungsgemäßen Verfahrens wird der Entwässerungsschritt a) in einem Gradientenmischer durchgeführt. Hierzu wird eine Gewebeprobe in ein Mischgefäß (Gradientenmischer) eingebracht. Es wird über einen Eingang ein entwässerndes Lösungsmittel eingebracht bis ein Restwassergehalt von 5 bis 20 Vol.-% erreicht ist. Das entwässernde Lösungsmittel kann eine Lösungsmittelkonzentration von 100 Vol.-% haben oder einen Restwassergehalt zwischen 2 und 50 Vol-% aufweisen. In einer Variante wird die Gewebeprobe im Gradientmischer zu Beginn direkt in ein entwässerndes Lösungsmittel mit einer Lösungsmittelkonzentration von 50 Vol.-% eingebracht und der Gradient dann in kleinen Schritten erhöht.

Bevorzugt wird die Gewebeprobe direkt aus dem Gradientenmischer in das Einbettungsmedium überführt, ohne dass es vorher in hochreinem Lösungsmittel inkubiert wird. Ein Inkubierungsschritt in hochreinem Lösungsmittel findet bevorzugt in Schritt a) und Schritt b) nicht statt.

Es ist überraschend, dass eine Klärung der Gewebeproben auf diese Weise möglich ist. Nach dem Stand der Technik läuft das Verfahren in einem Gradientenmischer wie folgt ab: Eine Gewebeprobe wird in ein Mischgefäß (Gradientenmischer) eingebracht. Dieses hat z.B. einen Eingang oben, und einen Ausgang seitlich. Wenn die Probe in 50% Ethanol in dieses Mischgefäß eingelegt wird und über den Eingang mit einer Dosierpumpe mit hoher Konzentration (> 95 Vol.-% Lösungsmittel) eingebracht wird, dann baut sich ein Gradient auf, der asymptotisch den Wert von 100 Vol.-% zu erreichen versucht. Auf diese Weise wird der Probe ein vorteilhafter stetig ansteigenden Ethanolgehalt angeboten. Da bei der Mischung die 100% nie erreicht werden kann, wird die Probe üblicherweise nachträglich noch in hochreinem Ethanol inkubiert. Nach dem Stand der Technik wird dieser Inkubationsschritt mehrfach wiederholt. Daher kann die Dosierpumpe in der Praxis aus kostentechnischen Gründen mit technischem Ethanol befüllt werden.

Erfindungsgemäß ist 1. der letzte (gegebenenfalls wiederholte) Inkubationsschritt mit absolutem Ethanol überflüssig geworden und 2. ist der mit dem Gradientenmischer erreichte Endpunkt, so lange er über etwa 80% Ethanol liegt, für die weitere Klärung der Gewebeprobe in Schritt b) ausreichend.

In einer weiteren alternativen Ausführungsform wird der Entwässerungsschritt a) in einem einzigen Durchgang durchgeführt. Es wird keine Entwässerungsreihe mit mehreren Durchgängen verwendet, sondern die Entwässerung gemäß Schritt a) erfolgt in einem einzigen Durchgang durch Einlegen der Gewebeprobe in entwässerndes Lösungsmittel mit einer Lösungsmittelkonzentration von mindestens 70 Vol.-%. Diese Ausführungsform eignet sich besonders für Gewebeproben mit kompaktem Gewebe, die wenig Wasser aufnehmen. Im nachfolgenden Schritt b) wird die Gewebeprobe sofort in das Einbettungsmedium gegeben, wiederum ohne Inkubation in einem hochreinen Lösungsmittel zur vollständigen Entwässerung. Dieses führt zu einem deutlichen Zeitgewinn und gleichzeitig auch zu einer deutlichen Einsparung bei den verwendeten Reagenzien. Die Einsparung bei den Reagenzien ergibt sich dabei zum einen aus der geringeren Menge Lösungsmittel, die benötigt wird. Zum anderen ist es nicht mehr notwendig ein hochreines wasserfreies Lösungsmittel einzusetzen, sondern es kann ein Lösungsmittel in technischer Qualität verwendet werden. Bei Ethanol muss beispielsweise nicht teurer 100% hochreiner Ethanol verwendet werden, sondern es kann der deutlich kostengünstigere vergällte Ethanol verwendet werden. Nach dem Stand der Technik wird die Inkubation mit hochreinem wasserfreien Lösungsmittel wiederholt, um die letzten Reste Wasser vollständig zu entfernen, auch wird in vielen Protokollen die Inkubation in dem Einbettungsmedium aus dem gleichen Grund wiederholt. Da erfindungsgemäß in beiden Ausführungsformen die abschließenden Inkubationen zur sicheren Entfernung von Restwasser aus dem Gewebe entfallen, werden gemäß des erfindungsgemäßen Verfahrens Reagenzien eingespart.

In einer Ausführungsform des erfindungsgemäßen Verfahrens wird die Gewebeprobe, bevor sie in Schritt a) dehydriert und Schritt b) optisch geklärt wird,
- fixiert und/oder
- mit Formaldehyd fixiert und/oder
- gewaschen und/oder
- mit Wasser gewaschen und/oder
- in einer wässrigen alkalischen Lösung mit nicht-ionischem Detergens inkubiert.
   und/oder
- mit einer Detergenzienlösung delipidiert (Delipidierung) und/oder
- mit einem organischen Lösungsmittel delipidiert (Delipidierung) und/oder
- mit oxidierenden Reagenzien gebleicht und/oder
- mit Aminoalkoholen entfärbt.

In einer Ausführungsform des erfindungsgemäßen Verfahrens wird die Gewebeprobe, bevor sie in Schritt a) dehydriert und Schritt b) optisch geklärt wird, fixiert und das Fixierungsmittel ausgewählt aus
- vernetzenden Fixantien wie Formaldehyd, Glutaraldehyd, Acrolein, Carbodiimide, Diethylpyrocarbonat, Bisimodo-Ester oder Glyoxal oder Mischungen hiervon, und/oder
- koagulierenden Fixantien wie Alkoholen und anderen organischen Lösungsmitteln, Säuren, Kaliumdichromat, Bleinitrat, Kupfersulfat und Quecksilberchlorid und Mischungen hiervon.

Das erfindungsgemäße Verfahren wird bevorzugt nach Gewebevorbereitungsschritten und nach Elektrophorese-Schritten durchgeführt. Die im erfindungsgemäßen Verfahren behandelte Gewebeprobe ist somit bereits vorbehandelt. Die Vorbehandlung durch Elektrophorese erfolgt bevorzugt gemäß dem in DE 10 2016 123 458 B3 beschriebenen Verfahren zur elektrophoretischen Klärung. Für die Durchführung der elektrophoretischen Klärung wird Bezug genommen auf die Patentschrift DE 10 2016 123 458 B3, deren Inhalt hiermit in diese Anmeldung aufgenommen wird.

Die optisch geklärte Gewebeprobe wird im erfindungsgemäßen Verfahren bevorzugt in einem weiteren Schritt unter einem Mikroskop untersucht, um ein Bild der inneren Struktur der Probe zu erhalten, wobei das Mikroskop ein Lichtmikroskop, bevorzugt ein Lichtscheibenmikroskop, ein Konfokal-Mikroskop, ein Zwei-Photonen-Mikroskop oder ein optisches Projektionstomographie-Mikroskop (OPT) ist.

Das bisher verwendete Einbettungsmedium Dibenzylether (DBE) weist einen Brechungsindex von n = 1.562 auf. Der zweite Reinstoff Ethylcinnamat besitzt einen Brechungsindex von n = 1.559. Von den erfindungsgemäßen Benzaldeyd-Anisol-Ethern besitzt z. B. 3-Methoxybenzaldehyd einen Brechungsindex von n = 1.552 und 2-Hydroxy-5-methoxybenzaldehyd eine Brechungsindex von n = 1.580. Bei der Verwendung der erfindungsgemäßen Benzaldehyd-Anisol-Ether als Einbettungsmedium für Gewebeclearing von entwässerten Gewebeproben konnte eine zumindest identische Durchsichtigkeit, teilweise eine verbesserte Durchsichtigkeit gegenüber Gewebeproben erzielt werden, die mit Methylsalicylat/Benzylbenzoat und mit Ethylcinnamat behandelt wurden.

Die Arbeiten von Spalteholz lassen bereits vermuten, dass sich die Brechungsindices unterschiedlicher Gewebe leicht voneinander unterscheiden. In seiner Originalveröffentlichung von 1911 wird folgende, an Hand des optimalen Mischungsverhältnisses von Methylsalicylat und BB empirisch bestimmten Reihe menschlicher Gewebe mit aufsteigendem Brechungsindex aufgeführt: junge Embryonen (5:1-3:1 je nach Gewicht) < erwachsene entkalkte Knochen (5:3) n = 1.547 < erwachsene Muskulatur ungefähr wie ältere Embryonen (2:1) < Gehirn und Rückenmark (1:1). Diese Variabilität hat direkte Folgen für die Durchsichtigkeit der unterschiedlichen Gewebe und deren mikroskopischen Untersuchung und insbesondere für quantitativ-spektroskopische Bestimmungen, welche mit einem Mikroskop durchgeführt werden können. Das erfindungsgemäße Verfahren bietet den Vorteil, dass verschiedene Reinstoffe als Einbettungsmedium eingesetzt werden, die je nach Gewebe ausgewählt werden können, um sich dem Brechungsindex des Gewebes anzunähern.

Gegenüber den bisher üblicherweise benutzten Einbettungsmedien Benzylbenzoat/Benzylalkohol-Mischungen (BABB) und Methylsalicylat-BenzylBenzoat-Mischungen besitzen die Benzaldehyd-Anisol-Ether somit den Vorteil, dass sie, genau wie Dibenzylether, als reine Substanz eingesetzt werden können: Da bereits die reine Substanz den gewünschten Brechungsindex erreicht, muss der Brechungsindex nicht durch Anmischen des Einbettungsmediums eingestellt werden. Somit entfällt ein Mischschritt und damit ein überflüssig gewordener Arbeitsschritt und eine mögliche Fehlerquelle.

Überraschenderweise dringen die erfindungsgemäßen Benzaldehyd-Anisol-Ether deutlich schneller in das entwässerte Gewebe ein als die bisher bekannten Einbettungsmedien und machen das Gewebe schnell durchsichtig. Bei der Vorbereitung der Probe lässt sich hierdurch Zeit einsparen und die Probe kann schneller der Untersuchung unter dem Lichtmikroskop zugeführt werden. Entsprechend liegen auch schneller die Ergebnisse der lichtmikroskopischen Untersuchungen vor. Die Dichte des entwässerten Gewebes wird durch die Dichte des noch im Gewebe enthaltenen entwässernden Lösungsmittels, z.B. Ethanol bestimmt. Da das Einbettungsmedium typischerweise eine höhere Dichte aufweist, ist die vollständige Eindringung des Einbettungsmediums in die Gewebeprobe gut durch das Absinken der Gewebeprobe im Einbettungsmedium nachzuverfolgen. Beispielweise wird mit 2-Hydroxy-5-methoxybenzaldehyd bereits nach 30-45 min ein vollständiges Eindringen des Einbettungsmediums in die Gewebeprobe erreicht d.h. die Gewebeproben liegen auf dem Gefäßboden. Bei herkömmlichen Einbettungsmedien wie Dibenzylether oder und Methylsalicylat-BenzylBenzoat-Mischungen sind dagegen typischerweise mehrere Stunden bis über Nacht für diesen Verfahrensschritt üblich. Das erfindungsgemäße Verfahren bietet somit den Vorteil, dass das Einbettungsmedium schneller in das Gewebe der Gewebeprobe diffundiert und bei der Vorbereitung der Probe eine deutliche Zeitersparnis erreicht wird. Die Gewebeproben können somit schneller lichtmikroskopisch untersucht werden.

Wenn die Gewebeprobe Restwasser enthält und in ein organisches Lösungsmittel als Einbettungsmedium überführt wird, in dem sich dieses Wasser nicht mehr lösen kann, wird das Wasser im Gewebe gefangen und führt auf Grund der unvollständigen Mischbarkeit zu einer die Durchsichtigkeit beeinträchtigenden Turbidität. Die Gewebeproben werden deshalb bisher z.B. üblicherweise mehrfach mit hoch-reinem Ethanol und oft anschließend wiederholt mit Einbettungsmedium inkubiert, um eine Beeinträchtigung der Transparenz der Probe zu vermeiden. Nach dem Stand der Technik wird deshalb, wie bereits erläutert, die Inkubation mit hochreinem wasserfreien Lösungsmittel wiederholt, um die letzten Reste Wasser vollständig zu entfernen, auch wird in vielen Protokollen die Inkubation in dem Einbettungsmedium aus dem gleichen Grund wiederholt.

Erfindungsgemäß erfolgt die Klärung des Gewebes mit einem Benzaldehyd-Anisol-Ether. Es hat sich überraschenderweise gezeigt, dass eine gute Transparenz auch bei Gewebeproben erreicht werden kann, die Restwasser enthalten. Bei kleinen Restwasseranteilen von 2-5 Vol.-% ist keinerlei Beeinträchtigung der Transparenz zu beobachten, und selbst bei hohen Wasseranteilen von bis zu 20 % bzw. bis zu 30 Vol.-%, abhängig von der behandelten Gewebeart, wird nur eine sehr geringe Turbidität beobachtet, die noch eine lichtmikroskopische Untersuchung der Probe zulässt.

Das erfindungsgemäße Verfahren bietet daher ein besonders schnelles und einfaches Verfahren zur Entwässerung einer Gewebeprobe, da anders als bei allen bisher bekannten Einbettungsmedien ein geringer Restwasseranteil bis zu 10 Vol.-% keine Beeinträchtigung der lichtmikroskopischen Ergebnisse bedingt und auch größere Restwassermengen noch zu einer akzeptablen Transparenz der Gewebeprobe führen.

Das erfindungsgemäße Verfahren erlaubt auch eine vereinfachte Verfahrensführung gegenüber Klärungsverfahren mit herkömmlichen Einbettungsmedien. Gemäß Stand der Technik wird die Gewebeprobe beim Entwässern von Gefäß zu Gefäß überführt mit einer dabei steigenden Konzentration des entwässernden Lösungsmittels, also z.B. mit einer steigenden Ethanolkonzentration. Es werden also recht viele Einzelschritte nötig und die Schritte sind diskret. Außerdem muss im letzten Schritt bzw. den letzten Schritten immer hochreines Lösungsmittel z.B. hochreiner Ethanol eingesetzt werden.

Beim erfindungsgemäßen Verfahren kann auch ein entwässerndes Lösungsmittel mit einem Restanteil an Wasser verwendet werden. So kann z.B. vergällter 95-97 %-iger technischer Ethanol anstelle des hochreinen Ethanols eingesetzt werden. Das erfindungsgemäße Verfahren führt daher auch zu einer Kosteneinsparung bei den eingesetzten Lösungsmitteln.

Das erfindungsgemäße Verfahren erlaubt auch eine vereinfachte Verfahrensführung bei Verwendung eines Gradientmischers, weil hier ebenfalls keine anschließende vollständige Entwässerung mit einem hochreinen Lösungsmittel erfolgen muss.

Die Benzaldehyd-Anisol-Ether weisen zudem eine geringe Toxizität auf. Dieses ist bei der Benutzung als Einbettungsmedium vorteilhaft, da sich die Gewebeprobe im Mikroskop unmittelbar unterhalb der Atemwege des Benutzers befindet und dieser von der Probe aufsteigende Dämpfe einatmen kann. Die Toxizität von para-Anisaldehyd ist laut der Europäische Chemikalienagentur ECHA toxikologisch unbedenklich, anders als Dibenzylether oder Benzylbenzoat/Benzylalkohol-Mischungen.

Das erfindungsgemäße Kit zur Präparation von biologischen Gewebeproben für die Lichtmikroskopie, enthält:
- ein entwässerndes Lösungsmittel zum Entwässern der Gewebeprobe und
- ein Einbettungsmedium zum Klären der entwässerten Gewebeprobe durch Einlegen in das Einbettungsmedium,
wobei
das Einbettungsmedium ein Benzaldehyd-Anisol-Ether, wie vorstehend beschrieben, ist, ausgewählt aus 3-Methoxybenzaldehyd, 4-Methoxybenzaldehyd, 2-Hydroxy-5-methoxybenzaldehyd und 4-Ethoxy-benzaldehyd. Der Benzaldehyd-Anisol-Ether wird bevorzugt in den vorstehend beschriebenen Konzentrationen und Reinheiten eingesetzt.

Das entwässernde Lösungsmittel im Kit ist ein Ether, Keton oder Alkohol, bevorzugt ist das Lösungsmittel ausgewählt aus Ethanol, Methanol, Isopropanol, tert. Butanol, 2,2' Thiodiethanol, Trichloroethanol, Tetrahydrofuran und Aceton.

Erfindungsgemäß erfolgt die Verwendung eines Benzaldehyd-Anisol-Ethers
ausgewählt aus 3-Methoxybenzaldehyd, 4-Methoxybenzaldehyd, 2-Hydroxy-5-methoxybenzaldehyd oder 4-Ethoxy-benzaldehyd
als Einbettungsmedium für die Präparation einer biologischen insbesondere humanen Gewebeprobe für die lichtmikroskopische Untersuchung.

Der erfindungsgemäß verwendete Benzaldehyd-Anisol-Ether wird bevorzugt in den vorstehend beschriebenen Konzentrationen und Reinheiten verwendet.

### Beispiele

### Beispiel 1

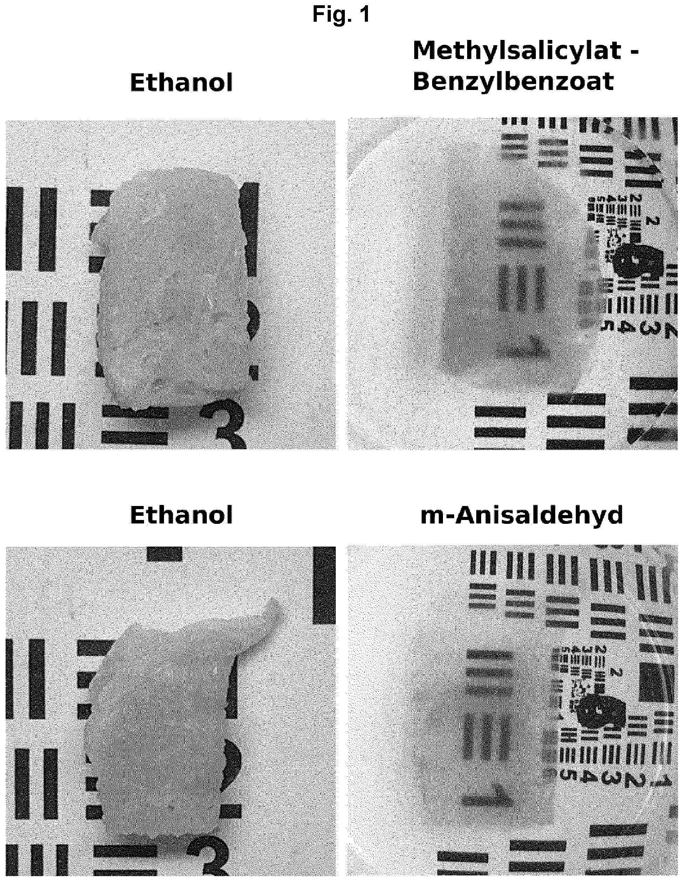

Es wurden mehrere Gewebeproben (Schweinelunge) mit einem Volumen von etwa 0,25 ml (0,5x0,5x1 cm³) in absolutem Ethanol entwässert und fotografiert. Die mit Ethanol entwässerten Gewebeproben sind in Figur 1, linke Seite dargestellt.

Die entwässerten Gewebeproben wurden dann für 3 Stunden in 5 ml des jeweiligen Einbettungsmediums gegeben. Als Einbettungsmedien wurden erfindungsgemäß 2-Hydroxy-5-methoxybenzaldehyd und als Vergleichsbeispiel ein Gemisch von Methylsalicylat mit Benzylbenzoat mit dem 2-Hydroxy-5-methoxybenzaldehyd angeglichenen Brechungsindex verwendet. Das Resultat des Klärungsschritts wurde ebenfalls fotografiert. Die Ergebnisse sind in Figur 1, rechte Seite dargestellt. Es zeigt sich, dass zwar bereits die Klärung mit Methylsalicylat/Benzylbenzoat zu einer guten Durchsichtigkeit der Gewebeprobe führt. Mit dem erfindungsgemäßen 2-Hydroxy-5-methoxybenzaldehyd konnte aber eine noch bessere Transparenz erzielt werden.

### Beispiel 2

Es wurden mehrere Gewebeproben (Schweinelunge) mit einem Volumen von etwa 0,25 ml (0,5x0,5x1 cm³) wie in Beispiel 1 beschrieben, vorbereitet, wobei als Einbettungsmedien 2-Hydroxy-5-Methoxybenzaldehyd, EthylCinnamat und Dibenzylether verwendet wurden.

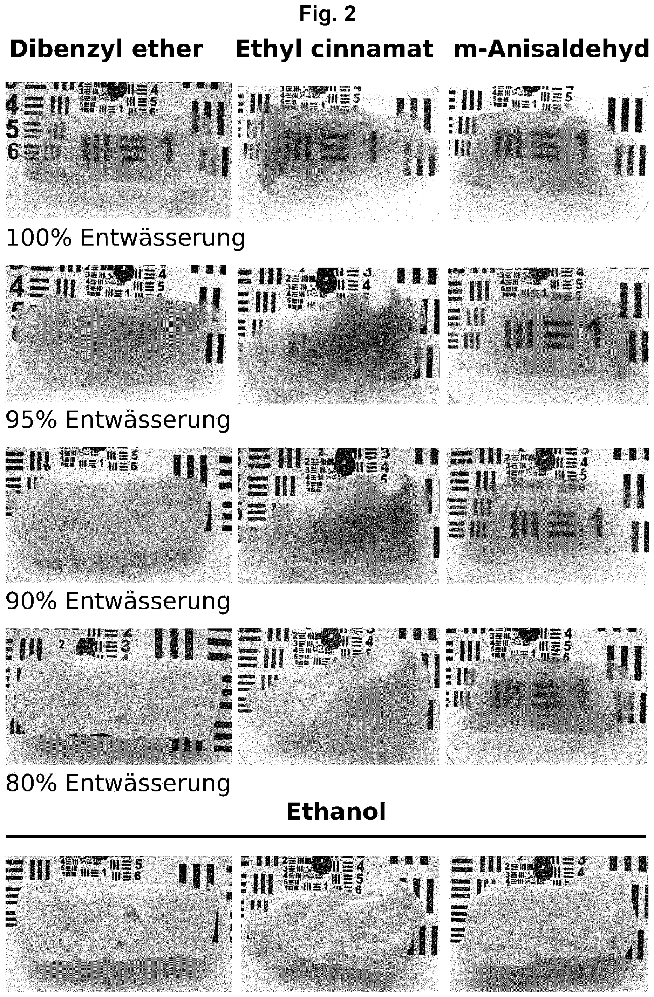

Dann wurden die Gewebeproben in absolutem Ethanol über Nacht gelagert und das Einbettungsmedium so wieder aus den Gewebeproben rausgewaschen. Die Gewebeproben wurden in Ethanol mit definierten Wassergehalten überführt, um einen definierten Restwasseranteil im Gewebe zu erreichen, 2 Stunden inkubiert und anschließend wieder in das jeweilige Einbettungsmedium gegeben, um fotografiert zu werden. Die Gewebeproben wurden in Ethanol mit 95 Vol.-% Ethanolgehalt, 90 Vol.-% Ethanolgehalt und 80 Vol.-% Ethanolgehalt gegeben. Die Resultate wurden fotografiert und sind in Figur 2 dargestellt. Die erste Reihe zeigt die zu 100 % entwässerten Gewebeproben in den Einbettungsmedien, die zweite Reihe zeigt die in Ethanol mit 5 Vol.-% Wassergehalt inkubierten Gewebeproben im Einbettungsmedium, die dritte Reihe zeigt die in Ethanol mit 10 Vol.-% Wassergehalt inkubierten Gewebeproben im Einbettungsmedium und die vierte Reihe zeigt die in Ethanol mit 20% Wassergehalt inkubierten Gewebeproben im Einbettungsmedium. Die untere Reihe zeigt die gleichen Gewebeproben in absolutem Ethanol, d.h. bevor sie in die jeweiligen Einbettungsmedien gegeben werden. Vollständig entwässert in Ethanol sind alle Proben gleichermaßen undurchsichtig.

Es zeigt sich, dass die aus dem Stand der Technik bekannten Einbettungsmedien Dibenzylether und Ethylcinnamat nur bei keinem Restwasser oder nur einer sehr geringen Restwassermenge die gewünschte Transparenz erreichen. Bei DBE reicht bereits eine Restwassermenge von 5 Vol.-% aus, damit die Probe nicht mehr durchsichtig wird und daher nicht mikroskopiert werden kann. Auch ECi zeigt bei einer Restwassermenge von 95 % schon eine deutliche Eintrübung, und die Gewebeprobe ist nichtmehr vollständig durchsichtig. Die mit 2-Hydroxy-5-Methoxybenzaldehyd geklärten Gewebeproben bleiben hingegen selbst bei einer Restwassermenge von 10 Vol.-% (90 Vol.-% Ethanol) vollständig durchsichtig und zeigen erst bei 20 % Restwasser (80% Ethanolgehalt) eine leichte Eintrübung.

### Beispiel 3

Gewebeproben mit einem Volumen von etwa 0,25 ml (0,5x0,5x1 cm³) wurden in absolutem Ethanol entwässert. Anschließend wurden sie in ein Einbettungsmedium in einem Probengefäß überführt. Als Einbettungsmedien wurden erfindungsgemäß 2-Hydroxy-5-methoxybenzaldehyd und als Vergleichsbeispiel Dibenzylether und Mischungen von Methylsalicylat mit Benzylbenzoat verwendet. Es konnte beobachtet werden, dass bei 2-Hydroxy-5-methoxybenzaldehyd die Gewebeprobe schon nach 15 min deutlich unter der Oberfläche hing und abhängig von der Art des Gewebes nach 30-45 min auf dem Boden des Gefäßes lag. In Dibenzylether sank die Gewebeprobe erst nach mehreren Stunden zu Boden, vergleichbar mit der Zeit, die Mischungen von Methylsalicylat/Benzylbenzoat und DBE benötigen.

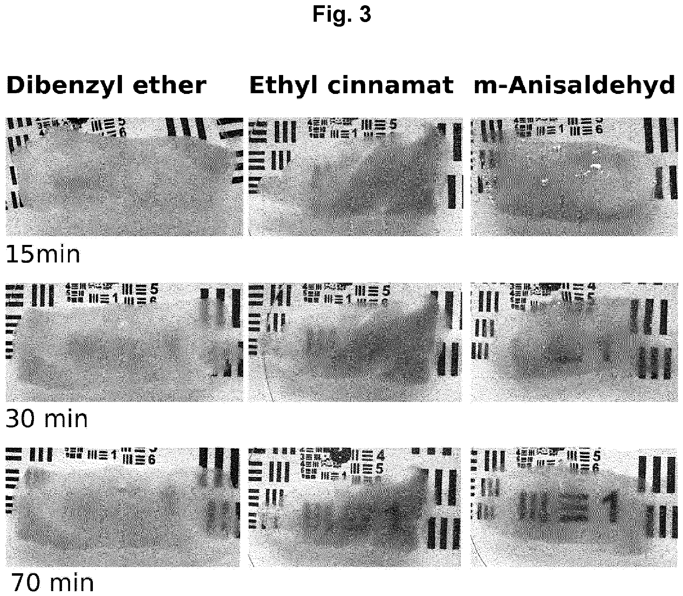

In einem weiteren Versuch wurden die Geschwindigkeit bestimmt, mit der das entwässernde Lösungsmittel Ethanol in einer Gewebeprobe durch die Einbettungsmedien ersetzt wurde. Als Einbettungsmedien wurden das erfindungsgemäße 2-Hydroxy-5-methoxybenzaldehyd und als Vergleichsbeispiele Dibenzylether und Ethylcinnemat verwendet. Die Gewebeproben wurden für 15 min, 30 min und 70 min in das Einbettungsmedium gelegt und dann fotografiert. Die Ergebnisse sind in Figur 3 dargestellt. In der oberen Reihe sind die Gewebeproben nach 15 min Klärung fotografiert. In der mittleren Reihe sind die Gewebeproben nach 30 min Klärung fotografiert. In der unteren Reihe sind die Gewebeproben nach 70 min Klärung fotografiert. Es ist zu erkennen, dass die mit 2-Hydroxy-5-methoxybenzaldehyd behandelte Gewebeprobe bereits nach 70 Min durchsichtig ist, während die mit ECi behandelte Probe nur eine leichte Durchsichtigkeit zeigt und die mit DBE behandelte Gewebeprobe noch gar nicht durchsichtig ist. Mit dem erfindungsgemäßen Klärungsverfahren wird somit eine deutlich schnellere Klärung der Gewebeprobe erreicht.

Die Erfindung ist nicht auf eine der vorbeschriebenen Ausführungsformen beschränkt, sondern in vielfältiger Weise abwandelbar.

Sämtliche aus den Ansprüchen, der Beschreibung und der Zeichnung hervorgehenden Merkmale und Vorteile, einschließlich Verfahrensschritten, können sowohl für sich als auch in den verschiedensten Kombinationen erfindungswesentlich sein.

## Patentansprüche

1. Verfahren zur Herstellung transparenter Gewebeprobe eines biologischen Gewebes zur lichtmikroskopischen Untersuchung, umfassend die Schritte:
a) Entwässern der Gewebeprobe mit einem entwässernden Lösungsmittels und
b) Klären der entwässerten Gewebeprobe durch Einlegen in ein Einbettungsmedium enthaltend einen Benzaldehyd-Anisol-Ether ausgewählt aus 3-Methoxybenzaldehyd, 4-Methoxybenzaldehyd, 2-Hydroxy-5-methoxybenzaldehyd oder 4-Ethoxy-benzaldehyd.

2. Verfahren gemäß Patentanspruch 1 **dadurch gekennzeichnet, dass** das Einbettungsmedium 10 bis 100 Vol.-% des Benzaldehyd-Anisol-Ethers enthält und 0 bis 90 Vol.-% eines optisch geeigneten, inerten organischen Lösungsmittels mit einem Brechungsindex von etwa 1.3, bevorzugt 1.5 enthält.

3. Verfahren gemäß Patentanspruch 1 **dadurch gekennzeichnet, dass** das Einbettungsmedium 10 bis 100 Vol.-% des Benzaldehyd-Anisol-Ethers enthält und 0 bis 90 Vol.-% eines optisch geeigneten, inerten organischen Lösungsmittels mit einem Brechungsindex von etwa 2.0, bevorzugt 1.65 enthält.

4. Verfahren gemäß einem der vorhergehenden Ansprüche, **dadurch gekennzeichnet, dass** das Einbettungsmedium aus einem Benzaldehyd-Anisol-Ether ausgewählt aus 3-Methoxybenzaldehyd, 4-Methoxybenzaldehyd, 2-Hydroxy-5-methoxybenzaldehyd oder 4-Ethoxy-benzaldehyd besteht.

5. Verfahren gemäß einem der vorhergehenden Ansprüche, **dadurch gekennzeichnet, dass** der Entwässerungsschritt a) die Anwendung von Dehydratisierungs-Zusammensetzungen aus wässrigem Alkohol, Keton oder Ether umfasst und die Dehydratisierungs-Zusammensetzungen eine wässrige Mischung eines Alkohols, Ketons oder Ethers mit einer Lösungsmittelkonzentration der Dehydratisierungs-Zusammensetzungen von 50 Vol.-% bis 98 Vol-%, bevorzugt 70 Vol.-% bis 98 Vol.-% , besonders bevorzugt 75 bis 98 Vol.% ist, bevorzugt eine wässrige Mischung aus wässrigem Ethanol mit steigenden Konzentrationen von Ethanol, wobei die Ethanolkonzentrationen der Dehydratisierungs-Zusammensetzungen von 50 Vol.-% bis 98 Vol-%reichen.

6. Verfahren gemäß einem der vorhergehenden Ansprüche, **dadurch gekennzeichnet, dass** der Entwässerungsschritt Schritt a) in einem Gradientenmischer durchgeführt wird und die Gewebeprobe bevorzugt zu Beginn des Entwässerungsschritts direkt in ein entwässerndes Lösungsmittel mit einer Lösungsmittelkonzentration von 50 % eingebracht und der Gradient dann in kleinen Schritten erhöht wird.

7. Verfahren gemäß einem der vorhergehenden Ansprüche, **dadurch gekennzeichnet, dass** die Gewebeprobe, bevor sie in Schritt a) dehydriert und Schritt b) optisch geklärt wird,
• fixiert wird und/oder
• mit Formaldehyd fixiert wird und/oder
• gewaschen wird und/oder
• mit Wasser gewaschen wird und/oder
• in einer wässrigen alkalischen Lösung inkubiert wird und/oder
• mit einer Detergenzienlösung delipidiert wird und/oder
• mit einem organischen Lösungsmittel delipidiert wird und/oder
• mit oxidierenden Reagenzien gebleicht wird und/oder
• mit Aminoalkoholen entfärbt wird.

8. Verfahren gemäß einem der vorhergehenden Ansprüche, **dadurch gekennzeichnet, dass** die Gewebeprobe, bevor sie in Schritt a) dehydriert und Schritt b) optisch geklärt wird, fixiert wird und das Fixierungsmittel ausgewählt ist aus
- vernetzenden Fixantien wie Formaldehyd, Glutaraldehyd, Acrolein, Carbodiimide, Diethylpyrocarbonat, Bisimodo-Ester oder Glyoxal oder Mischungen hiervon, und/oder
- koagulierenden Fixantien wie Alkoholen und anderen organischen Lösungsmitteln, Säuren, Kaliumdichromat, Bleinitrat, Kupfersulfat und Quecksilberchlorid und Mischungen hiervon.

9. Verfahren gemäß einem der vorhergehenden Ansprüche, **dadurch gekennzeichnet, dass** die optisch geklärte Gewebeprobe in einem weiteren Schritt unter einem Mikroskop untersucht wird, um ein Bild der inneren Struktur der Probe zu erhalten, wobei das Mikroskop ein Lichtmikroskop ist.

10. Kit zur Präparation von biologischen Gewebeproben für die Lichtmikroskopie, enthaltend:
- ein entwässerndes Lösungsmittel zum Entwässern der Gewebeprobe und
- ein Einbettungsmedium zum Klären der entwässerten Gewebeprobe durch Einlegen in das Einbettungsmedium,
wobei das entwässernde Lösungsmittel ausgewählt ist aus Ethanol, Isopropanol oder tert. Butanol und in Form von Dehydratisierungs-Zusammensetzungen aus wässrigem Lösungsmittel mit steigenden Konzentrationen von Lösungsmittel vorliegt,
und wobei
das Einbettungsmedium ein Benzaldehyd-Anisol-Ether ausgewählt aus 3-Methoxybenzaldehyd, 4-Methoxybenzaldehyd, 2-Hydroxy-5-methoxybenzaldehyd oder 4-Ethoxy-benzaldehyd ist.

11. Kit gemäß Patentanspruch 10, **dadurch gekennzeichnet, dass** der Benzaldehyd-Anisol-Ether ausgewählt ist aus 3-Methoxybenzaldehyd, 4-Methoxybenzaldehyd, 2-Hydroxy-5-methoxybenzaldehyd und 4-Ethoxy-benzaldehyd.

12. Verwendung Benzaldehyd-Anisol-Ethers ausgewählt aus 3-Methoxybenzaldehyd, 4-Methoxybenzaldehyd, 2-Hydroxy-5-methoxybenzaldehyd oder 4-Ethoxy-benzaldehyd
als Einbettungsmedium für die Präparation einer biologischen Gewebeprobe für die lichtmikroskopische Untersuchung.

## Claims

1. Method for producing a transparent tissue sample of a biological tissue for light-microscopic examination, comprising the steps of:
a) dehydrating the tissue sample with a dehydrating solvent and
b) clearing the dehydrated tissue sample by placing it into an embedding medium containing a benzaldehyde anisole ether selected from 3-methoxybenzaldehyde, 4-methoxybenzaldehyde, 2-hydroxy-5-methoxybenzaldehyde or 4-ethoxybenzaldehyde.

2. Method according to Claim 1, **characterized in that** the embedding medium contains 10% to 100% by volume of the benzaldehyde anisole ether and contains 0% to 90% by volume of an optically suitable, inert organic solvent having a refractive index of about 1.3, preferably 1.5.

3. Method according to Claim 1, **characterized in that** the embedding medium contains 10% to 100% by volume of the benzaldehyde anisole ether and contains 0% to 90% by volume of an optically suitable, inert organic solvent having a refractive index of about 2.0, preferably 1.65.

4. Method according to any of the preceding claims, **characterized in that** the embedding medium consists of a benzaldehyde anisole ether selected from 3-methoxybenzaldehyde, 4-methoxybenzaldehyde, 2-hydroxy-5-methoxybenzaldehyde or 4-ethoxybenzaldehyde.

5. Method according to any of the preceding claims, **characterized in that** the dehydration step a) comprises the use of dehydrating compositions composed of aqueous alcohol, ketone or ether and the dehydrating compositions are an aqueous mixture of an alcohol, ketone or ether having a solvent concentration of the dehydrating compositions from 50% by volume to 98% by volume, preferably 70% by volume to 98% by volume, particularly preferably 75% to 98% by volume, preferably an aqueous mixture of aqueous ethanol having increasing concentrations of ethanol, where the ethanol concentrations of the dehydrating compositions range from 50% by volume to 98% by volume.

6. Method according to any of the preceding claims, **characterized in that** the dehydration step a) is carried out in a gradient mixer and the tissue sample is introduced preferably at the start of the dehydration step directly into a dehydrating solvent having a solvent concentration of 50% and the gradient is then increased in small increments.

7. Method according to any of the preceding claims, **characterized in that** the tissue sample, before it is dehydrated in step a) and optically cleared in step b),
• is fixed and/or
• is fixed with formaldehyde and/or
• is washed and/or
• is washed with water and/or
• is incubated in an aqueous alkaline solution and/or
• is delipidated with a detergent solution and/or
• is delipidated with an organic solvent and/or
• is bleached with oxidizing reagents and/or
• is decolorized with amino alcohols.

8. Method according to any of the preceding claims, **characterized in that** the tissue sample, before it is dehydrated in step a) and optically cleared in step b), is fixed and the fixing agent is selected from
- crosslinking fixatives such as formaldehyde, glutaraldehyde, acrolein, carbodiimides, diethyl pyrocarbonate, bisimido esters or glyoxal or mixtures thereof, and/or
- coagulating fixatives such as alcohols and other organic solvents, acids, potassium dichromate, lead nitrate, copper sulfate and mercury chloride and mixtures thereof.

9. Method according to any of the preceding claims, **characterized in that** in a further step the optically cleared tissue sample is examined under a microscope in order to obtain an image of the internal structure of the sample, where the microscope is a light microscope.

10. Kit for preparing biological tissue samples for light microscopy, containing:
- a dehydrating solvent for dehydrating the tissue sample and
- an embedding medium for clearing the dehydrated tissue sample by placing the sample into the embedding medium,
where the dehydrating solvent is selected from ethanol, isopropanol or tert-butanol and is in the form of dehydrating compositions composed of aqueous solvent with increasing concentrations of solvent,
and where
the embedding medium is a benzaldehyde anisole ether selected from 3-methoxybenzaldehyde, 4-methoxybenzaldehyde, 2-hydroxy-5-methoxybenzaldehyde or 4-ethoxybenzaldehyde.

11. Kit according to Claim 10, **characterized in that** the benzaldehyde anisole ether is selected from 3-methoxybenzaldehyde, 4-methoxybenzaldehyde, 2-hydroxy-5-methoxybenzaldehyde and 4-ethoxybenzaldehyde.

12. Use of a benzaldehyde anisole ether selected from 3-methoxybenzaldehyde, 4-methoxybenzaldehyde, 2-hydroxy-5-methoxybenzaldehyde or 4-ethoxybenzaldehyde as an embedding medium for preparing a biological tissue sample for light-microscopic examination.

## Revendications

1. Procédé de fabrication d'échantillons de tissus transparents d'un tissu biologique pour examen au microscope optique, comprenant les étapes :
a) déshydratation de l'échantillon de tissu avec un solvant déshydratant et
b) clarification de l'échantillon de tissu déshydraté par introduction dans un milieu d'enrobement contenant un alcoxybenzaldéhyde choisi parmi 3-méthoxybenzaldéhyde, 4-méthoxybenzaldéhyde, 2-hydroxy-5-méthoxybenzaldéhyde ou 4-éthoxy-benzaldéhyde.

2. Procédé selon la revendication 1, **caractérisé en ce que** le milieu d'enrobement contient 10 à 100 % en volume de l'alcoxybenzaldéhyde et 0 à 90 % en volume d'un solvant organique inerte optiquement approprié ayant un indice de réfraction d'environ 1,3, de préférence de 1,5.

3. Procédé selon la revendication 1, **caractérisé en ce que** le milieu d'enrobement contient 10 à 100 % en volume de l'alcoxybenzaldéhyde et 0 à 90 % en volume d'un solvant organique inerte optiquement approprié ayant un indice de réfraction d'environ 2,0, de préférence de 1,65.

4. Procédé selon l'une des revendications précédentes, **caractérisé en ce que** le milieu d'enrobement est constitué d'un alcoxybenzaldéhyde choisi parmi 3-méthoxybenzaldéhyde, 4-méthoxybenzaldéhyde, 2-hydroxy-5-méthoxybenzaldéhyde ou 4-éthoxy-benzaldéhyde.

5. Procédé selon l'une des revendications précédentes, **caractérisé en ce que** l'étape de déshydratation a) comprend l'utilisation de compositions déshydratantes constituées d'un alcool, d'une cétone ou d'un éther en solution aqueuse, et **en ce que** les compositions déshydratantes sont un mélange aqueux d'un alcool, d'une cétone ou d'un éther, la concentration du solvant dans les compositions déshydratantes étant de 50 % en volume à 98 % en volume, de préférence de 70 % en volume à 98 % en volume, d'une manière particulièrement préférée de 75 à 98 % en volume, de préférence un mélange aqueux d'éthanol aqueux présentant des concentrations croissantes d'éthanol, les concentrations de l'éthanol dans les compositions déshydratantes atteignant 50 % en volume à 98 % en volume.

6. Procédé selon l'une des revendications précédentes, **caractérisé en ce que** l'étape de déshydratation a) est mise en œuvre dans un mélangeur à gradient, et l'échantillon de tissu est de préférence au début de l'étape de déshydratation introduit directement dans un solvant déshydraté présentant une concentration du solvant de 50 %, le gradient pouvant ensuite être augmenté par petites étapes.

7. Procédé selon l'une des revendications précédentes, **caractérisé en ce que** l'échantillon de tissu, avant d'être déshydraté dans l'étape a) et optiquement clarifié dans l'étape b), est
• fixé et/ou
• fixé avec du formaldéhyde et/ou
• lavé et/ou
• lavé à l'eau et/ou
• incubé dans une solution alcaline aqueuse et/ou
• délipidé avec une solution d'un détergent et/ou
• délipidé avec un solvant organique et/ou
• blanchi avec des réactifs oxydants et/ou
• décoloré avec des amino-alcools.

8. Procédé selon l'une des revendications précédentes, **caractérisé en ce que** l'échantillon de tissu, avant d'être déshydraté dans l'étape a) et optiquement clarifié dans l'étape b), est fixé, et l'agent de fixation est choisi parmi
- les fixatifs réticulables tels que le formaldéhyde, le glutaraldéhyde, l'acroléine, les carbodiimides, le pyrocarbonate de diéthyle, les bisimido-esters ou le glyoxal ou les mélanges de ceux-ci, et/ou
- les fixatifs coagulables tels que les alcools et d'autres solvants organiques, les acides, le bichromate de potassium, le nitrate de plomb, le sulfate de cuivre, le chlorure de mercure et les mélanges de ceux-ci.

9. Procédé selon l'une des revendications précédentes, **caractérisé en ce que** l'échantillon de tissu optiquement clarifié est, dans une autre étape, examiné au microscope, pour obtenir une image de la structure interne de l'échantillon, le microscope étant un microscope optique.

10. Trousse pour la préparation d'échantillons de tissus biologiques pour microscopie optique, contenant :
- un solvant déshydratant pour déshydrater l'échantillon de tissu et
- un milieu d'enrobement pour clarifier l'échantillon de tissu déshydraté par introduction dans le milieu d'enrobement,
le solvant déshydratant étant choisi parmi l'éthanol, l'isopropanol ou le tert-butanol et se présentant sous forme de compositions déshydratantes d'un solvant aqueux présentant des concentrations croissantes du solvant, et le milieu d'enrobement étant un alcoxybenzaldéhyde choisi parmi 3-méthoxybenzaldéhyde, 4-méthoxybenzaldéhyde, 2-hydroxy-5-méthoxybenzaldéhyde ou le 4-éthoxy-benzaldéhyde.

11. Trousse selon la revendication 10, **caractérisée en ce que** l'alcoxybenzaldéhyde est choisi parmi 3-méthoxybenzaldéhyde, 4-méthoxybenzaldéhyde, 2-hydroxy-5-méthoxybenzaldéhyde et 4-éthoxy-benzaldéhyde.

12. Utilisation d'un alcoxybenzaldéhyde choisi parmi 3-méthoxybenzaldéhyde, 4-méthoxybenzaldéhyde, 2-hydroxy-5-méthoxybenzaldéhyde ou 4-éthoxy-benzaldéhyde, comme milieu d'enrobement pour la préparation d'un échantillon biologique pour examen au microscope optique.
